(19) Europäisches Patentamt
European Patent Office
Office européen des brevets

(11) Veröffentlichungsnummer: 0 364 808 A2

(12) # EUROPÄISCHE PATENTANMELDUNG

(21) Anmeldenummer: 89118299.0

(51) Int. Cl.$^5$: C07C 37/20

(22) Anmeldetag: 03.10.89

(30) Priorität: 15.10.88 DE 3835204

(43) Veröffentlichungstag der Anmeldung: 25.04.90 Patentblatt 90/17

(84) Benannte Vertragsstaaten: BE DE ES FR GB IT NL

(71) Anmelder: BAYER AG
D-5090 Leverkusen 1 Bayerwerk(DE)

(72) Erfinder: Bottenbruch, Ludwig, Dr.
Woehlerstrasse 5
D-4150 Krefeld(DE)
Erfinder: Ruppert, Heinrich, Dr.
Pappelweg 21
D-5060 Bergisch Gladbach 2(DE)
Erfinder: Isaacs, Neil Stewart, Prof. Dr.
Rosewood Gallowstree Road
Peppard Henley on Thames, RG5 9ET(GB)
Erfinder: Latham, Kenneth Geoffrey, Dr.
Curtoys Westbrook Street
Blewsbury Berkshire, OX11 9QB(GB)

(54) **Verfahren zur Herstellung von Dihydroxidiarylalkanen.**

(57) Die Erfindung betrifft ein Verfahren zur Herstellung von Dihydroxidiarylalkanen aus Carbonylverbindungen und Phenolen unter hohem Druck.

EP 0 364 808 A2

## Verfahren zur Herstellung von Dihydroxidiarylalkanen

Die Erfindung betrifft ein Verfahren zur Herstellung von Dihydroxidiarylalkanen aus Carbonylverbindungen und Phenolen unter hohem Druck.

Dihydroxidiarylalkane sind bekannt. Sie werden im allgemeinen durch Kondensation aromatischer Hydroxiverbindungen mit Aldehyden oder Ketonen hergestellt. Das bekannteste Dihydroxidiarylalkan ist 4,4′-Dihydroxidiphenyl-2,2-propan. Man erhält es aus Phenol und Aceton gemäß

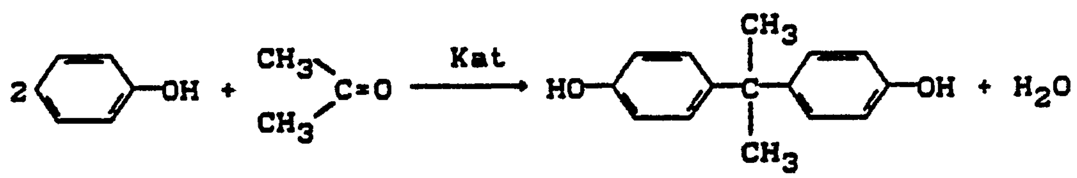


Das Produkt wird meist Kurz als Bisphenol-A (BPA) bezeichnet (A steht dabei für Aceton).

Die Reaktion von Phenol und Aceton kann z.B. durch Säuren katalysiert werden. Dabei können starke Säuren, z.B. Chlorwasserstoff, Schwefelsäuren, Sulfonsäuren bzw. saure Ionenaustauscher oder auch Aluminiumchlorid, Zinkchlorid etc. verwendet werden.

Die technische Umsetzung liefert nicht reines 4,4′-Dihydroxidiphenyl-2,2-propan, sondern ein Gemisch, das erhebliche Mengen (bis 20 %) an Nebenprodukten und Isomeren enthalten kann (z.B. Ullmann, 4. Aufl. Bd. 18, S 217 ff).

Man kann die Reaktion von Phenolen und Aceton in Substanz oder in inerten organischen Lösungsmitteln durchführen. Um z.B. reines, handelsfähiges Bisphenol-A zu erhalten, muß das primäre Reaktionsgemisch aufgearbeitet werden. Dazu sind mehrere Stufen, u.a. auch zur Entfernung von Nebenprodukten und gegebenenfalls Katalysatorresten, erforderlich (Schnell, Chemistry and Physics of Polycarbonates, Interscience Publishers 1964, S 82/83).

Bei ca. 30° liegt die Zeit bis zur vollständigen Umsetzung der Reaktionspartner bei ca. 12-24h. Die Reaktionszeit läßt sich durch erhöhte Temperatur (50-60°C) auf etwa ein Drittel abkürzen; trotzdem erfordert die technische Herstellung von BPA noch immer sehr große Reaktionsgefäße. Außerdem erhöht sich bei erhöhter Temperatur auch der Anteil der Nebenprodukte (z.B. o, -p-Isomeren); er beträgt bei 60°C ca. 7-10%.

Es wurde nun gefunden, daß die Reaktion von Phenolen und Aceton, gegebenenfalls in Gegenwart von Katalysatoren, z.B. zum Bisphenol A, bei Raumtemperatur unter Drucken von 1000 bis 10 000 bar nur wenige Minuten in Anspruch nimmt. Dabei wird in hoher Ausbeute und praktisch ohne Nebenprodukte z.B. reines 4,4′-Dihydroxydiphenyl-2,2-propan erhalten.

Die technische Bedeutung dieser Erfindung liegt darin, daß die Reaktion ein sehr geringes Reaktionsvolumen benötigt und z.B. in dünnen Röhren ausgeführt werden kann. Außerdem entfällt die aufwendige Abtrennung der Nebenprodukte und gegebenenfalls der organischen Schwefelverbindungen.

Das Verfahren läßt sich mit gutem Erfolg neben Aceton auf andere Ketone (z.B. Methylethylketon, Cyclohexanon etc.) und Phenole (z.B. Kresol, z.B. Dimethylphenol) übertragen.

Gegenstand der Erfindung ist daher ein Verfahren zur Herstellung von 4,4′-Dihydroxydiphenylalkanen, vorzugsweise von 4,4′-Dihydroxidiphenylpropan und seinen in den aromatischen Kernen durch $C_1$-$C_3$-Alkylgruppen, vorzugsweise Methylgruppen oder durch Halogen, vorzugsweise Cl, Br, substituierten Derivaten, dadurch gekennzeichnet, daß man einen Überschuß der Phenole mit Ketonen, vorzugsweise Aceton bei einem Druck von mindestens 1000 bar, gegebenenfalls in Anwesenheit eines Katalysators und gegebenenfalls in Anwesenheit eines wasserbindenden Mittels, bei Temperaturen von 20 bis 100°C umsetzt.

Insbesondere verwendet man für das Verfahren mindestens 3 Mol Phenol pro Mol Keton, vorzugsweise Aceton. Als Phenol kann dieses selbst und z.B. o-Kresol, o-Chlorphenol, 2,6-Dimethylphenol, o-Ethylphenol, o-Isopropylphenol, o-Phenylphenol u.s.w., vorzugsweise Phenol eingesetzt werden.

Bevorzugt werden 30 bis 3 Mol des Phenols pro Mol Keton, vorzugsweise Aceton verwendet.

Die Reaktion wird unter hohem Druck ausgeführt. Mindestens wendet man einen Druck von 1000 bar an. Die obere Druckgrenze ist nur durch die technischen Möglichkeiten gegeben und dürfte in der Praxis derzeit bei etwa 10 000 bis 15 000 bar liegen. Bevorzugt ist ein Druck von ca. 3000 bis ca. 6000 bar, besonders bevorzugt um 5000 bar, weil sich dort optimale Ergebnisse mit vertretbarem technischen Aufwand erreichen lassen. Durch die Anwendung der hohen Drucke wird die Reaktion erheblich beschleunigt (bis zu einem Faktor von $10^6$), die Ausbeute erhöht und der Anteil an 4,4′-Dihydroxidiphenyl-Verbindungen im Reaktionsprodukt gesteigert.

Die Reaktion kann in grundsätzlich bekannter Weise mit den für diesen Zweck bekannten basischen und sauren Katalysatoren und Co-Katalysatoren beschleunigt werden.

Als Katalysator können Lewis-Säuren wie $ZnCl_2$, Protonensäuren wie Toluolsulfonsäure, Chloressigsäure, Wasser, Halogenwasserstoffe wie Chlorwasserstoff, Trifluoressigsäure, Bortrifluorid (Etherat) u.s.w. eingesetzt werden.

Gasförmige Katalysatoren sind bevorzugt (z.B. HCl).

Geeignete Katalysatormengen sind im allgemeinen 0,01 bis 20 Gew.-%, bevorzugt 1 bis 10 Gew.-%, besonders bevorzugt 4 bis 6 Gew.-%, bezogen auf das Phenol.

Es können auch $SO_3H$-Gruppen enthaltende Ionenaustauscher oder Methansulfonsäure verwendet werden.

Der Katalysator kann auch SH-Gruppen enthalten, z.B. mit Mercaptoverbindungen belegte $SO_3H$-Gruppen enthaltende Ionenaustauscher.

Als Co-Katalysatoren könne SH-Gruppen haltige Substanzen verwendet werden.

Die erfindungsgemäße Reaktion kann ohne oder mit Lösungsmittel durchgeführt werden. Als Lösungsmittel kommen sämtliche, bei den Reaktionsbedingungen inerten organischen Flüssigkeiten in Frage, z.B. Halogenkohlenwasserstoffe, bevorzugt Chlorkohlenwasserstoffe. Bei Mitverwendung eines Lösungsmittels wird die spätere Aufarbeitung des Reaktionsgemisches erleichtert. Ohne Lösungsmittel wird im allgemeinen eine höhere Ausbeute erzielt.

Als Ionenaustauscherharze können handelsübliche Harze, z.B. sulfoniertes Polystyrol wie IR 120® u.s.w. verwendet werden.

Das erfindungsgemäße Verfahren kann kontinuierlich oder diskontinuierlich durchgeführt werden. Bei der diskontinuierlichen Arbeitsweise werden Gemische der Ausgangsprodukte und gegebenenfalls Katalysator, Entwässerungsmittel, Lösungsmittel in einem geeigneten Autoklaven unter Druck umgesetzt, das Reaktionsprodukt entnommen und in an sich bekannter Weise, z.B. durch Destillation bzw. Kristallisation, aufgearbeitet. Bei der kontinuierlichen Arbeitsweise benutzt man einen Rohrreaktor, in dem die Reaktanten kontinuierlich eingepreßt werden, den sie durchlaufen und an dessen Ende sie kontinuierlich entnommen werden.

Das erfindungsgemäße Verfahren ergibt 4,4′-Dihydroxidiphenyl-2,2-propane in hohen Ausbeuten und in größer Reinheit. Wegen der geringen Anteile an Nebenprodukt ist die Aufarbeitung vereinfacht und wegen der Schnelligkeit der Umsetzung und der guten Ausbeuten sind die Reaktionszeiten stark verkürzt und damit die Raum/Zeit-Ausbeuten erheblich verbessert.

## Beispiele

Die Herstellung der Bisphenole erfolgt in für diesen Zweck bekannten Apparaturen analog bekannten Verfahrensweisen.

Es können beispielsweise 1,5 m lange und 3 cm Innendurchmesser aufweisende Reaktionsrohre verwendet werden.

Bei der Durchführung der Umsetzung werden die eingesetzten Katalysatoren vorzugsweise separat gegebenenfalls in einem Lösungsmittel zur Carbonylverbindung und zum Phenol dosiert. Wird Phenol im Überschuß eingesetzt, kann nicht umgesetztes Phenol abdestilliert werden.

Bei Verwendung eines Ionenaustauschharzes als Katalysator werden Phenol und Carbonylverbindung gegebenenfalls in Mischung über das Harz unter den erfindungsgemäßen Bedingungen geleitet.

| Beispiel | mol-Verhältnis Phenol-Keton | Katalysator Gew.-% | p/kbar | T/°C | t/min | Ausbeute |
|---|---|---|---|---|---|---|
| 1 | 3:1 | 4 % HCl | 5.5 | 25 | 15 | 80 |
| 2 | 3:1 | 4 % HCl | 5.5 | 25 | 10 | 83 |
| 3 | 3:1 | 4 % HCl | 0.001 | 0-10 | 180 | 30 |
| 4 | 3:1 | 4 % HCl | 0.001 | -18 | 3Tage | 10 |
| 5 | 3:1 | 4 % HCl | 5.5 | 25 | 10 | 75 |
| 6 | 3:1 | 4 % HCl | 5.5 | 40 | 10 | 82 |
| 7 | 3:1 | 4 % HCl | 5.5 | 60 | 10 | 90 |
| 8 | 3:1 | 4 % HCl | 3 | 25 | 10 | 45 |
| 9 | 3:1 | 4 % HCl | 5 | 25 | 10 | 71 |
| 10 | 3:1 | 4 % HCl | 6.5 | 25 | 10 | 74 |
| 11 | 3:1 | 3,4 % HCl | 5 | 25 | 30 | 65 |
| 12 | 4:1 | 2,7 % HCl | 5 | 50 | 10 | 90 |
| 13 | 3:1 | 4 % $tolSO_3H$ | 5 | 25 | 105 | 2 |
| 14 | 3:1 | 4 % ClAcOH | 5 | 25 | 105 | 1 |
| 15 | 3:1 | 4 % AcOH | 5 | 25 | 105 | 0 |
| 16 | 8:1 | 2,4 % HCl | 5 | 25 | 30 | 72 |
| 17 | 8:1 | 2,4 % HCl | 5 | 50 | 30 | 91 |
| 18 | 3:1 | 4 % TFA | 5 | 25 | 105 | 0 |
| 19 | 3:1 | 4 % DABCO | 5 | 25 | 105 | 0 |
| 20 | 3:1 | 4 % PhONa | 5 | 25 | 105 | 0 |
| 21 | 3:1 | 4 % TFA | 5 | 25 | 960 | 0 |
| 22 | 3:1 | 4 % TFA* | 5 | 25 | 105 | 0 |
| 23 | 3:1 | 4 % TFA | 5 | 60 | 30 | 0 |
| 24 | 3:1 | 4 % $tolSO_3H$ | 5 | 60 | 30 | 15 |
| 25 | 3:1 | 4 % aq.TFA | 5 | 60 | 105 | 0 |

| Beispiel | mol-verhalten Phenol-Keton | Katalysator Gew.-% | p/kbar | T/°C | t/min | Ausbeute |
|---|---|---|---|---|---|---|
| 26 | 3:1 | 4 % Wasser | 5 | 60 | 105 | 0 |
| 27A | 3:1 | 5 % MSA | 5 | 60 | 30 | 64 |
| 27B | 3:1 | 10 % MSA | 5 | 60 | 30 | 80 |
| 27C | 3:1 | 15 % MSA | 5 | 60 | 30 | 83 |
| 28 | 3:1 | resin | 5 | 60 | 30 | 95 |
| 29 | 3:1 | 10 % MSA | 5 | 60 | 10 | 78 |
| 30 | 3:1 | 4 % $BF_3$ | 5 | 60 | 30 | 64 |
| 31 | 3:1 | 12 % $ZnCl_2$ | 5 | 60 | 30 | 1 |
| 32 | 3:1 | 1 % HCl | 5 | 25 | 10 | 21 |
| 33 | 3:1 | 1 % HCl | 5 | 25 | 30 | 23 |
| 34 | 3:1 | 1 % HCl | 5 | 60 | 10 | 47 |
| 35 | 3:1 | 1 % HCl | 5 | 60 | 30 | 46 |
| 36 | 21.6:1 | 4 % MSA | 5 | 60 | 30 | 66 |
| 37 | 21.6:1 | 2 % MSA | 5 | 60 | 30 | 54 |
| 38 | 21.6:1 | 4 % MSA | 5 | 60 | 15 | 63 |
| 39 | 21.6:1 | 4 % MSA | 5 | 60 | 10 | 53 |
| 40 | 21.6:1 | 4 % MSA | 5 | 60 | 5 | 53 |
| 41 | 21.6:1 | 4 % MSA | 5 | 60 | 1 | 30 |
| 42 | 3:1 | 0,88 % HCl | 5 | 60 | 30 | 50 |
| 43 | 3:1 | 0,88 % HCl + 0,35 % BT | 5 | 60 | 30 | 99 |
| 44 | 3:1 | 2,7 % MSA | 5 | 60 | 30 | 25 |
| 45 | 3:1 | 2,7 % MSA + 2,0 % BT | 5 | 60 | 30 | 78 |
| 46 | 3:1 | 2,7 % MSA + trace $H_2S$ | 5 | 60 | 30 | 54 |

| | |
|---|---|
| HCl | Salzsäure |
| $tolSO_3H$ | Toluolsulfonsäure |
| ClAcOH | Chloressigsäure |
| TFA | Trifluoressigsäure |
| resin | Sulfoniertes Polystyrol |
| DABCO | Triethylendiamin (Diazabicyclooctan) |
| $BF_3$ | Bortrifluorid |
| MSA | Methansulfonsäure |
| BT | Benzylthiol |
| $H_2S$ | Schwefelwasserstoff |

## Ansprüche

1. Verfahren zur Herstellung von 4,4′-Dihydroxydiphenylalkanen, vorzugsweise von 4,4′-Dihydroxidiphenylpropan und seinen in den aromatischen Kernen durch $C_1$-$C_3$-Alkylgruppen, vorzugsweise Methylgruppen oder durch Halogen, vorzugsweise Cl, Br, substituierten Derivaten, dadurch gekennzeichnet, daß man einen Überschuß der Phenole mit Ketonen, vorzugsweise Aceton bei einem Druck von mindestens 1000 bar, gegebenenfalls in Anwesenheit eines Katalysators und gegebenenfalls in Anwesenheit eines wasserbindenden Mittels, bei Temperaturen von 20 bis 100 °C umsetzt.

2. Verfahren nach Anspruch 1, dadurch gekennzeichnet, daß man bei einem Druck von 3000 bis 6000 bar und einer Temperatur von 20 bis 60 °C arbeitet.

3. Verfahren nach Anspruch 1, dadurch gekennzeichnet, daß man als Katalysator Chlorwasserstoff oder einen $SO_3H$-Gruppen enthaltenden Ionenaustauscher oder Methansulfonsäure verwendet.

4. Verfahren nach Anspruch 1, dadurch gekennzeichnet, daß der Katalysator SH-Gruppen enthält.